# EUROPEAN PATENT APPLICATION

(11) **EP 4 318 184 A1**
(43) Date of publication of application: **07.02.2024**
(21) Application number: 21933050.3
(22) Date of filing: 25.03.2021
(51) Int. Cl.: G06F 3/01, G06F 3/0488

(54) **INFORMATION PROCESSING SYSTEM, INFORMATION PROCESSING METHOD, AND COMPUTER PROGRAM**

(71) Applicant: NEC Corporation, 108-8001 Tokyo (JP); NEC Platforms, Ltd., Kanagawa 213-8511 (JP)
(72) Inventor: INAGAKI, Fumiyuki, Kawasaki-shi, Kanagawa 213-8511 (JP); IRIE, Fumi, Tokyo 108-8001 (JP)
(74) Representative: Betten & Resch
(86) International application number: PCT/JP2021/012599
(87) International publication number: WO 2022/201442

(57) **Abstract**

An information processing system includes: a terminal used by a user; a detection unit that detects at least one of a position of contact by the user and a position in which droplets of saliva of the user are attached, in the terminal; and an identification unit that identifies a sanitization part to be sanitized of the terminal, on the basis of the at least one of the positions. According to such an information processing system, it is possible to properly identify the part to be sanitized in an operation terminal.

## Description

### Technical Field

This disclosure relates to technical fields of an information processing system including a terminal operated by a user, an information processing method, and a computer program.

### Background Art

A known system of this type deals with information about droplets of saliva. For example, Patent Literature 1 discloses a technique/technology of estimating a reachable range of the droplets from a position of a mouth and a direction of a face of a target person, or the like. Patent Literature 2 discloses a technique/technology of detecting the droplets by using an infrared camera. Patent Literature 3 discloses a technique/technology of identifying a part touched by a finger with the droplets thereon, as a contaminated area.

As another related technique/technology, for example, Patent Literature 4 discloses a technique/technology of detecting a generation position of an infectious substance and performing sanitization or sterilization. Patent Literature 5 discloses a technique/technology related to Biocart used in airports or the like.

### Citation List

### Patent Literature

Patent Literature 1: International Publication No. WO2020/044826
Patent Literature 2: International Publication No. WO2019/146456
Patent Literature 3: JP2018-084975A
Patent Literature 4: International Publication No. WO2020/059442
Patent Literature 5: Japanese Patent No. 6813209

### Summary

### Technical Problem

This disclosure aims to improve the techniques/technologies disclosed in Citation List.

### Solution to Problem

An information processing system according to an example aspect of this disclosure includes: a terminal used by a user; a detection unit that detects at least one of a position of contact by the user and a position in which droplets of saliva of the user are attached, in the terminal; and an identification unit that identifies a sanitization part to be sanitized of the terminal, on the basis of the at least one of the positions.

An information processing method according to an example aspect of this disclosure is an information processing method using a terminal used by a user, the information processing method including: detecting one of a position of contact by the user and a position in which droplets of saliva of the user are attached, in the terminal; and identifying a sanitization part to be sanitized of the terminal, on the basis of the at least one of the positions.

A computer program according to an example aspect of this disclosure is a computer program that allows a computer to execute an information processing method using a terminal used by a user, the information processing method including: detecting one of a position of contact by the user and a position in which droplets of saliva of the user are attached, in the terminal; and identifying a sanitization part to be sanitized of the terminal, on the basis of the at least one of the positions.

### Brief Description of Drawings

[FIG. 1] FIG. 1 is a block diagram illustrating a hardware configuration of an information processing system according to a first example embodiment.
[FIG. 2] FIG. 2 is a block diagram illustrating a functional configuration of the information processing system according to the first example embodiment.
[FIG. 3] FIG. 3 is a flowchart illustrating a flow of operation of the information processing system according to the first example embodiment.
[FIG. 4] FIG. 4 is a conceptual diagram illustrating an application example of an information processing system according to a second example embodiment.
[FIG. 5] FIG. 5 is a perspective view illustrating a specific configuration of an operation terminal in the information processing system according to the second example embodiment.
[FIG. 6] FIG. 6 is a block diagram illustrating a functional configuration of an information processing system according to a third example embodiment.
[FIG. 7] FIG. 7 is a flowchart illustrating a flow of operation of a baggage detection unit according to the third example embodiment.
[FIG 8] FIG. 8 is a block diagram illustrating a functional configuration of an information processing system according to a fourth example embodiment.
[FIG. 9] FIG. 9 is a flowchart illustrating a flow of operation of a specific movement detection unit according to the fourth example embodiment.
[FIG. 10] FIG. 10 is a block diagram illustrating a functional configuration of an information processing system according to a fifth example embodiment.
[FIG. 11] FIG. 11 is a flowchart illustrating a flow of operation of a temperature change detection unit according to the fifth example embodiment.
[FIG. 12] FIG. 12 is a block diagram illustrating a functional configuration of an information processing system according to a sixth example embodiment.
[FIG. 13] FIG. 13 is a flowchart illustrating a flow of operation of the information processing system according to the sixth example embodiment.
[FIG. 14] FIG. 14 is a block diagram illustrating a functional configuration of an information processing system according to a seventh example embodiment.
[FIG. 15] FIG. 15 is a flowchart illustrating a flow of operation of the information processing system according to the seventh example embodiment.
[FIG. 16] FIG. 16 is a block diagram illustrating a functional configuration of an information processing system according to an eighth example embodiment.
[FIG. 17] FIG. 17 isa flowchart illustrating a low of operation of the information processing system according to the eighth example embodiment.

### Description of Example embodiments

Hereinafter, an information processing system, an information processing method, and a computer program according to example embodiments will be described with reference to the drawings.

### <First Example Embodiment>

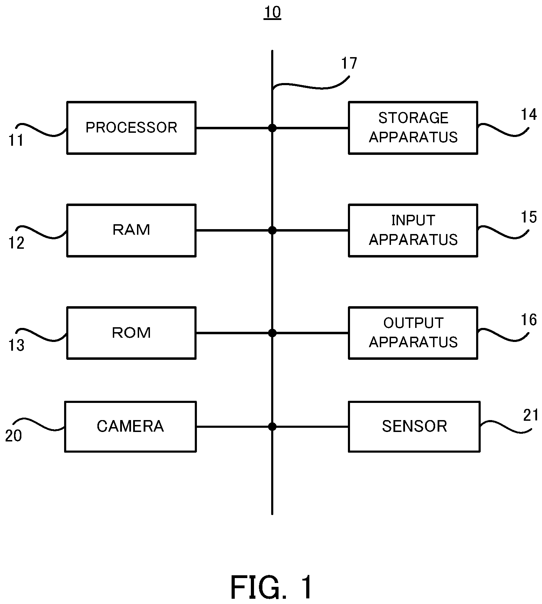

An information processing system according to a first example embodiment will be described with reference to FIG. 1 to FIG. 3.

### (Hardware Configuration)

First, a hardware configuration of an information processing system 10 according to the first example embodiment will be described with reference to FIG. 1. FIG. 1 is a block diagram illustrating the hardware configuration of the information processing system according to the first example embodiment.

As illustrated in FIG. 1, the information processing system 10 according to the first example embodiment includes a processor 11, a RAM (Random Access Memory) 12, a ROM (Read Only Memory) 13, and a storage apparatus 14. The information processing system 10 may further include an input apparatus 15 and an output apparatus 16. The information processing system 10 may include a camera 20 and a sensor 21. The processor 11, the RAM 12, the ROM 13, the storage apparatus 14, the input apparatus 15, the output apparatus 16, the camera 20 and the sensor 21 are connected through a data bus 17.

The processor 11 reads a computer program. For example, the processor 11 is configured to read a computer program stored by at least one of the RAM 12, the ROM 13, and the storage apparatus 14. Alternatively, the processor 11 may read a computer program stored in a computer-readable recording medium, by using a not-illustrated recording medium reading apparatus. The processor 11 may obtain (i.e., may read) a computer program from a not-illustrated apparatus disposed outside the information processing system 10, through a network interface. The processor 11 controls the RAM 12, the storage apparatus 14, the input apparatus 15, and the output apparatus 16 by executing the read computer program. Especially in this example embodiment, when the processor 11 executes the read computer program, a functional block for performing a process of identifying a sanitization part in a terminal, is realized or implemented in the processor 11. An example of the processor 11 includes a CPU (Central Processing Unit), a GPU (Graphics Processing Unit), a FPGA (field-programmable gate array), a DSP (Demand-Side Platform), and an ASIC (Application Specific Integrated Circuit). The processor 11 may use one of the examples described above, or may be use a plurality or them in parallel.

The RAM 12 temporarily stores the computer program to be executed by the processor 11. The RAM 12 temporarily stores the data that is temporarily used by the processor 11 when the processor 11 executes the computer program. The RAM 12 may be, for example, a D-RAM (Dynamic RAM).

The ROM 13 stores the computer program to be executed by the processor 11. The ROM 13 may otherwise store fixed data. The ROM 13 may be, for example, a P-ROM (Programmable ROM).

The storage apparatus 14 stores the data that is stored for a long term by the information processing system 10. The storage apparatus 14 may operate as a temporary storage apparatus of the processor 11. The storage apparatus 14 may include, for example, at least one of a hard disk apparatus, a magneto-optical disk apparatus, a SSD (Solid State Drive), and a disk array apparatus.

The input apparatus 15 is an apparatus that receives an input instruction from a user of the information processing system 10. The input apparatus 15 may include, for example, at least one of a keyboard, a mouse, and a touch panel. The input apparatus 15 may be a dedicated controller (operation terminal). The input apparatus 15 may include a terminal owned by the user (e.g., a smartphone, a tablet terminal, etc.). The input apparatus 15 may be an apparatus that allows an audio input including a microphone, for example.

The output apparatus 16 is an apparatus that outputs information about the information processing system10 to the outside. For example, the output apparatus 16 may be a display apparatus (e.g., a display) that is configured to display the information about the information processing system 10. The display apparatus here may be a TV monitor, a personal computer monitor, a smartphone monitor, a tablet terminal monitor, or another portable terminal monitor. The display apparatus may be a large monitor or a digital signage installed in various facilities such as stores. The output apparatus 16 may be an apparatus that outputs the information in a format other than an image. For example, the output apparatus 16 may be a speaker that audio-outputs the information about the information processing system 10.

### (Functional Configuration)

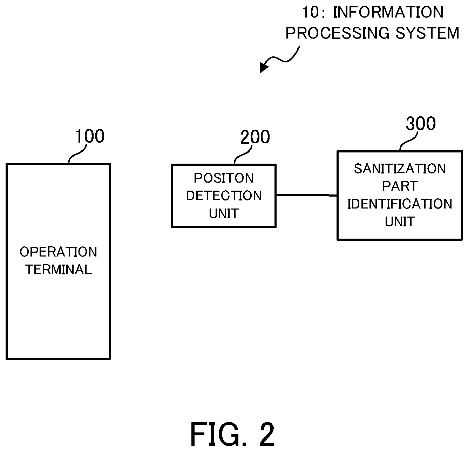

Next, a functional configuration of the information processing system 10 according to the first example embodiment will be described with reference to FIG. 2. FIG. 2 is a block diagram illustrating the functional configuration of the information processing system according to the first example embodiment.

As illustrated in FIG. 2, the information processing system 10 according to the first example embodiment includes an operation terminal 100, a position detection unit 200, and a sanitization part identification unit 300. Here, the operation terminal 100, the position detection unit 200 and the sanitization part identification unit 300 are separately illustrated, but the position detection unit 200 and the sanitization part identification unit 300 may be functions that are realized or implemented inside the operation terminal 100. Alternatively, the position detection unit 200 and the sanitization part identification unit 300 may be functions that are realized or implemented by an external server or cloud.

The operation terminal 100 is configured as a terminal that may be used by a plurality of users. The operation terminal is configured to receive an operation by a user, and to perform each process corresponding to the operation, for example. A specific example of the operation terminal will be described in detail in a second example embodiment described later.

The position detection unit 200 is configured to detect at least one of a position of contact by the user and a position in which droplets of saliva of the user are attached, in the operation terminal 100. The "position of contact by the user" here may include not only a position of direct contact by the user, but also a position of indirect contact by the user (e.g., a position of contact by a baggage of the user) or the like. In addition, the "position in which the droplets of saliva of the user are attached" may be a position in which the droplets of the user are estimated to be attached. A specific detection method by the position detection unit 200 will be described in detail in another example embodiment described later.

The sanitization part identification unit 300 is configured to identify a sanitization part (i.e., a part to be sanitized) in the operation terminal 100, on the basis of the position of contact by the user (hereinafter referred to as a "contact position" as appropriate) and the position in which the droplets of the user are attached (hereinafter referred to as a "droplet position" as appropriate), which are detected by the position detection unit 200. The sanitization part identification unit 300 may identify all of the contact positions and the droplet positions detected by the position detection unit 200, as the sanitization part, or may identify a part of the contact positions and the droplet positions, or surroundings of the contact positions and the droplet positions, as the sanitization part.

### (Flow of Operation)

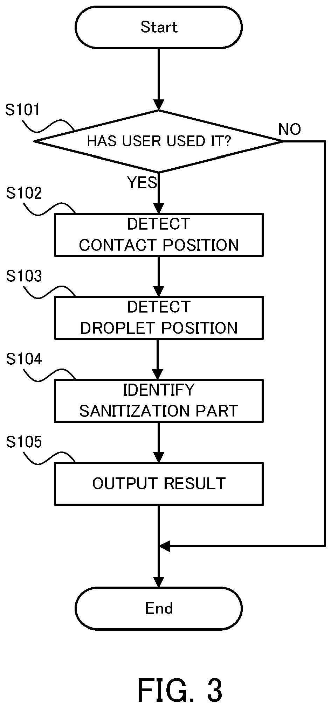

Next, a flow of operation of the information processing system 10 according to the first example embodiment will be described with reference to FIG. 3. FIG. 3 is a flowchart illustrating the flow of the operation of the information processing system according to the first example embodiment.

As illustrated in FIG. 3, in operation of the information processing system 10 according to the first example embodiment, it is first detected whether or not the user has used the operation terminal 100 (step S101). The use of the operation terminal 100 may be detected when the user is at a position to use the operation terminal 100, or may be detected when the user actually operates the operation terminal 100 (e.g., the user touches the operation terminal), for example. When it is determined that the user has not use the operation terminal 100 (step S101: NO), the subsequent process may be omitted.

When it is determined that the user has used the operation terminal 100 (step S101: YES), the position detection unit 200 detects the contact position (step S102). The position detection unit 200 also detects the droplet position (step S103). Only one of the step S102 and the step S103 may be performed. When the step S102 and the step S103 are both performed, the step S102 and step S103 may be performed before and after each other, or may be performed simultaneously in parallel.

Subsequently, the sanitization part identification unit 300 identifies the sanitization part of the operation terminal 100, on the basis of the contact position and the droplet position (step S104). Then, the sanitization part identification unit 300 outputs information about the identified sanitization part (step S105). The information about the sanitization part may be outputted, for example, by using the output apparatus 16.

### (Technical Effect)

Next, a technical effect obtained by the information processing system 10 according to the first example embodiment will be described.

As described in FIG. 1 to FIG. 3, in the information processing system 10 according to the first example embodiment, the sanitization part of the operation terminal 100 is identified on the basis of the contact position and the droplet position. In this way, it is possible to properly identify the part to be sanitized after the use by the user (i.e., the part that may be contaminated by the operation by the user). Therefore, it is possible to properly sanitize the part to be sanitized, so that it is possible to prevent the spread of infectious diseases, for example.

### <Second Example Embodiment>

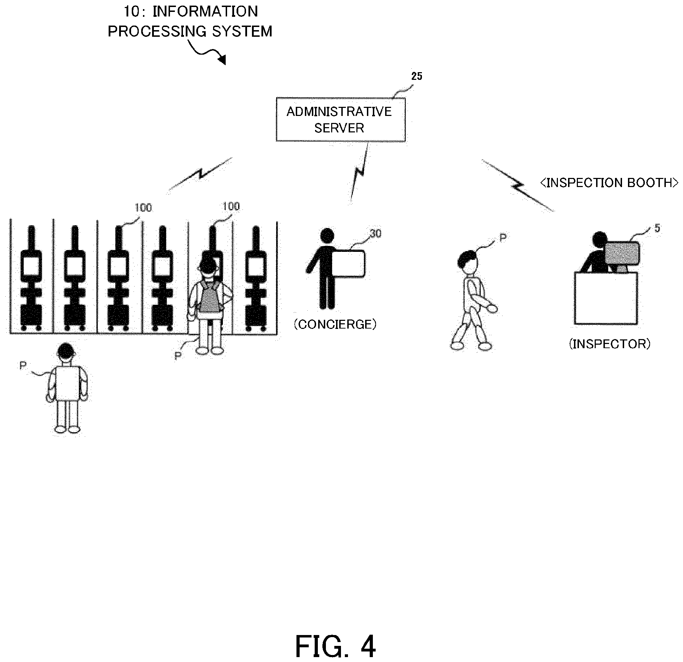

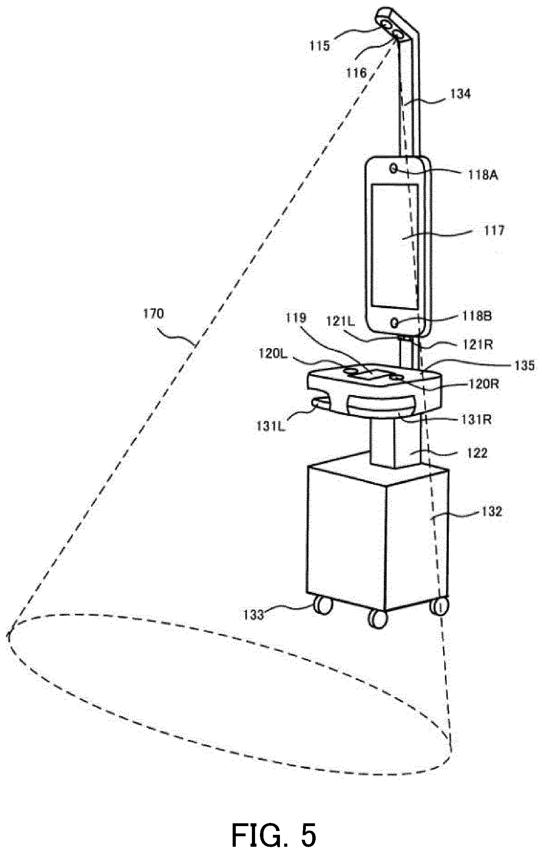

The information processing system 10 according to a second example embodiment will be described with reference to FIG. 4 and FIG. 5. The second example embodiment indicates a specific application example of the first example embodiment, and may be the same as the first example embodiment in the main configuration and operation (see FIG. 1 to FIG. 3). For this reason, a description of a part that overlaps with the first example embodiment described above will be omitted below.

### <Overall Configuration>

First, an overall configuration of the information processing system 10 according to the second example embodiment will be described with reference to FIG. 4. FIG. 4 is a conceptual diagram illustrating an application example of the information processing system according to the second example embodiment.

As illustrated in FIG. 4, the information processing system 10 according to the second example embodiment is configured as an immigration inspection system installed in an airport, for example. The immigration inspection is basically carried out in two steps: acquisition of personal identification information, and immigration inspection by an inspector. Specifically, when an entrant gets out of an airplane and enters an immigration inspection area, the entrant first operates the operation terminal 100 to provide the personal identification information. Then, the entrant goes to an inspection booth where the inspector is, and undergoes the inspection by the inspector. Upon completion of the inspection by the inspector, the entrant is permitted to enter a country.

The information processing system 10 according to the second example embodiment includes a plurality of operation terminals 100, an administrative server 25, a tablet 30, and a PC 5. The operation terminal 100 is provided to obtain the personal identification information on an entrant P. The entrant P may be for not only foreign nationals, but also all people who wish to enter the country. The entrant P may be not only a human, but also an animal, such as a dog, a cat, and a snake. These entrants P are an example of the "users." The personal identification information may include a face image or a fingerprint.

in the vicinity of the operation terminal 100, a concierge stands by to assist in management and operation of the operation terminal 100. A predetermined number of operation terminals 100 are assigned to the concierge to be in charge. The concierge has the tablet 30 that is used to manage the operation terminal 100 or perform similar operations. The tablet 30 is an example, and any terminal apparatus that allows the management of the operation terminals 100 may be used, such as a PC or a smartphone. An assistant (supporter) who carries out supplementary work may be assigned to the concierge.

At the inspection booth, the inspector stands by to conduct the inspection for the entrant P who has already provided the personal identification information. The inspection booth is provided with the PC 5 to be used by the inspector. The inspector displays the personal identification information on the entrant P, or the like, on the PC5 to conduct the inspection. FIG. 4 illustrates only one inspection booth for convenience, but a plurality of inspection booths are actually provided.

The administrative server 25 manages and controls the operation terminals 100 and the tablet 30, through wireless communication. The administrative servers 25 also communicate wiredly or wirelessly with the PC 5 used by the inspector. Specifically, the operation terminal 100 transmits the information provided by the entrant P who operates the operation terminal 100, to the administrative server 25. The administrative server 25 stores the information obtained from the operation terminal 100, and transmits the information to the tablet 30 and the PC 5. The tablet 30 receives information indicating an operation state of the operation terminal 100 or the like, from the administrative server 25, and displays the information. Thus, the concierge can understand the situation of the operation terminals 100 in charge.

In addition, the administration server 25 transmits the personal identification information provided by the entrant P who operates the operation terminal 100, to the PC 5 at the inspection booth. The inspector displays the personal identification information on the entrant P received from the control server 25, on the PC 5, and conducts the inspection by looking at the contents. In the above example, the operation terminal 100, the tablet 30 of the concierge, and the PC5 at the inspection booth transmit and receive the information through the administrative server 25; however, it may be configured such that the information is directly transmitted and received, without the administrative server 25, between the operation terminal 100 and the tablet 30, and between the operation terminal 100 and the PC 5 at the inspection booth.

### <Configuration of Operation Terminal>

Next, a specific configuration of the operation terminal 100 in the immigration inspection system described above will be described with reference to FIG. 5. FIG 5 is a perspective view illustrating the specific configuration of the operation terminal in the information processing system according to the second example embodiment.

As illustrated in FIG. 5, the operation terminal 100 includes a thermosensor 115, a monitor camera 116, a touch panel 117, two face authentication cameras 118Aand 118B, a passport reader 119, a pair of fingerprint scanners 120R and 120L, a pair of cameras close at hand 121R and 121L, a lifting mechanism 122, a pair of handles for movement 131R and 131L, a base 132, a moving casters 133, a bar 134, and a table 135. In the following description, subscripts are omitted when there is no need to specify individual elements that make a pair. For example, when any of the fingerprint scanners 120R and 120R is specified, it is referred to as the "fingerprint scanner 120R" and the "fingerprint scanner 120L", and if any of them is not specified, they are simply referred to as the "fingerprint scanners 120".

The thermosensor 115 is provided at the top of the operation terminal 100 and detects the approach of the user to the operation terminal 100. Basically, in a state in which the thermosensor 115 does not detect the user, the operation terminal 100 is in a standby (sleep) state. When the user stands in front of the thermosensor 115, the thermosensor 115 detects the user as a heat source, and the operation terminal 100 is activated.

The monitor camera 116 is provided at the top of the operation terminal 100, as in the thermosensor 115, and images a predetermined range in front of the operation terminal 100 from diagonally above. The monitor camera 116 is used to image the behavior or the like of the user in front of the operation terminal 100.

The touch panel 117 is provided at a position corresponding to an upper body of the user standing in front of the operation terminal 100, and is movable in a vertical direction along the bar 134. The touch panel 117 serves as an input unit and a display unit, when the user operates the operation terminal 100. The touch panel 117 may display guidance information required when the user operates the operation terminal 100. In addition, when the user needs to make some selection for the guidance information, a selection button may be displayed on the touch panel 117.

The face authentication camera 118A is provided at an upper end of the touch panel 117, and the face authentication camera 118B is provided at a lower end of the touch panel 117. Basically, the face authentication camera 118A at the upper end is used to capture a face image of a tall person, and the face authentication camera 118B at the lower end is used to capture a face image of a short person, such as a child. Thus, the selection of the face authentication camera to be used in accordance with an imaging target, increases the possibility of capturing an image suitable for the face authentication (e.g., a front face image). The face authentication camera 118A at the upper end may be used to capture the face image of a short person, or the face authentication camera 118B at the lower end may be used to capture the face image of a tall person. In addition, a plurality of face authentication cameras mat be used to obtain the face images captured at various angles as well as the front face image.

When the touch panel 117 is movable in the vertical direction along the bar 134 as in this example embodiment, a single face authentication camera may be provided near the center of the touch panel 117 in the vertical direction. Furthermore, in the example in FIG. 5, the face authentication cameras 118A and 118B are provided at the upper end and the lower end outside a display area of the touch panel 117, but instead, a half mirror may be provided on the touch panel 117 and the face authentication camera may be provided at any position inside the half mirror. By providing the face authentication camera inside the half mirror as described above, it is possible to capture the face image without making the user aware of the presence of the camera.

The table 135 is provided under the touch panel 117. The passport reader 119 and the pair of fingerprint scanners 120R and 120L are provided on an upper surface of the table 135. The passport reader 119 reads recorded information through wireless communication from an IC chip in a passport placed on the passport reader 119. Specifically, the passport reader 119 reads, from the passport, matters for identification such as nationality, name, date of birth, and passport number, and the face image of a photograph affixed to a passport application or the like (hereinafter collectively referred to as "passport information"). The fingerprint scanners 120 read fingerprints of the user's left and right index fingers. The fingerprint scanner 120R is for the right hand, and the fingerprint scanner 120L is for the left hand.

The lifting mechanism 122 moves the table 135 in the vertical direction. The lifting mechanisms 122 allow the table 135 to be moved to a level that matches the height of the user, i.e., a height that allows the user to easily place the fingers on the fingerprint scanners 120R and 120L. The lifting mechanism 122 also moves the touch panel 117 along the bar 134. Any mechanism that moves the touch panel 117 along the bar 134 may be used. For example, a rail may be provided on a front surface of the bar, the touch panel 117 may be fixed to a slider capable of moving up and down inside the rail, and the slider may be moved up and down by the lifting mechanism 122, so that the touch panel 117 may be moved up and down.

The pair of cameras close at hand 121R and121L are provided above the table 135. The cameras close at hand 121R and 121L image a state in which the user places the passport on the passport reader 119, a state in which the user puts the left and right fingers on the fingerprint scanners 120, and similar states. The camera close at hand 121R images the right hand side of the user, i.e., the fingerprint scanner 120R side, while the camera close at hand 121L images the left hand side of the user, i.e., the fingerprint scanner 120L side. The cameras close at hand 121 may be provided at the lower end of the touch panel 117, or may be provided on the bar 134 at a position behind the touch panel 117.

The base 132 is a housing provided at a lower end of the operation terminal 100, and a removable battery or spare battery or the like is housed therein. Since the operation terminal 100 is powered by the removable battery, the operation terminal 100 may be moved and used in a place without any outlet. The moving casters 133 are provided on the bottom of the base 132, and the pair of handles for movement 131R and 131L are provided at a lower end of the table 135. The concierge and other workers can move the operation terminal 100 with the handles for movement 131.

### <Method of Detecting Contact Position>

Next, a method of detecting the contact position in the operation terminal 100 described above (see FIG. 5) will be described with reference to FIG. 5.

The position detection unit 200 may detect the contact position of the user, by using the monitor camera 116, the face authentication cameras 118A and 118B, and the cameras close at hand 121R and 121L in the operation terminal 100. Specifically, the position detection unit 200 may detect the contact position of the user (e.g., a part touched by the user's hand, etc.), by analyzing the images captured by the monitor camera 116, the face authentication cameras 118A and 118B, and the cameras close at hand 121R and 121L.

The position detection unit 200 may detect the contact position of the user, by using the touch panel 117 in the operation terminal 100. Specifically, the position detection unit 200 may detect a part of the touch panel 117 touched by the user, as the contact position of the user.

The position detection unit 200 may detect the contact position of the user, by using a weight sensor (not illustrated) mounted in each part (e.g., the table 135 or the like) of the operation terminal 100. Specifically, the position detection unit 200 may detect a part in which a change in weight is detected by the weight sensor, as the contact position of the user.

### (Technical Effect)

Next, a technical effect obtained by the information processing system 10 according to the second example embodiment will be described.

As described in FIG. 4 and FIG. 5, in the information processing system 10 according to the second example embodiment, the contact position of the user is detected by using at least one of the various cameras (the monitor camera 116, the face authentication cameras 118A and 118B, and the cameras close at hand 121R and 121L), the touch panel 117, and the weight sensor, which are provided in the operation terminal 100. In this way, it is possible to accurately detect the contact position of the user. Furthermore, it is not necessary to provide a dedicated member for detecting the contact position (i.e., it is possible to utilize the member originally provided as the operation terminal 100), and thus, it is also possible to prevent an increase in cost.

The immigration inspection system described in the second example embodiment is merely one application example, and the information processing system 10 is also applicable to other systems. For example, the information processing system 10 may be applied to a system, such as a POS register system and ATM, operated by the user. In the following example embodiments, a description will be given on the premise that the operation terminal 100 is configured as in the second example embodiment (see FIG. 5).

<Third Example Embodiment>

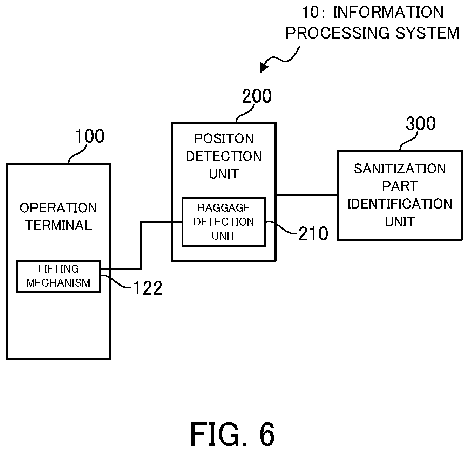

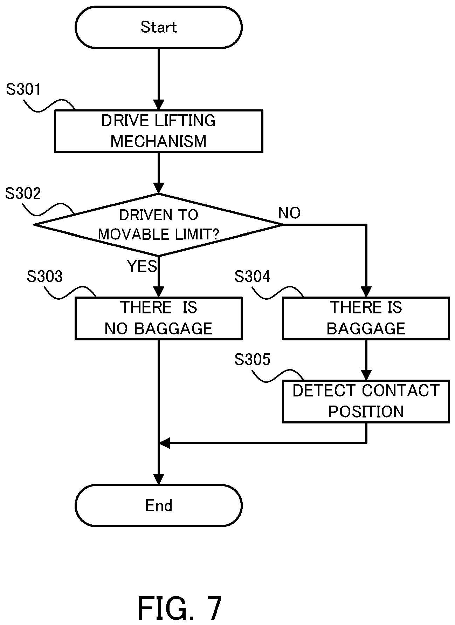

The information processing system 10 according to a third example embodiment will be described with reference to FIG. 6 and FIG. 7. The third example embodiment is partially different from the first and second example embodiments only in the configuration and operation, and may be the same as the first and second example embodiments in other parts. For this reason, a description of a part that overlaps with the example embodiments described above will be omitted below.

### (Functional Configuration)

First, a functional configuration of the information processing system 10 according to the third example embodiment will be described with reference to FIG. 6. FIG. 6 is a block diagram illustrating the functional configuration of the information processing system according to the third example embodiment. In FIG. 6, the same components as those illustrated in FIG. 2 carry the same reference numerals.

As illustrated in FIG. 6, the information processing system 10 according to the third example embodiment includes the operation terminal 100, the position detection unit 200, and the sanitization part identification unit 300. In particular, the position detection unit 200 according to the third example embodiment includes a baggage detection unit 210.

The baggage detection unit 210 is configured to detect a baggage of the user placed on the operation terminal 100, by using the lifting mechanism 122 in the operation terminal 100. For example, the baggage detection unit 210 uses the lifting mechanism 122 to drive the table 135 to move downward. At this time, for example, if the baggage of the user baggage is placed under the table 135 (i.e., on the base 132), the table 135 is stopped without going down to a movable limit. As described above, the baggage detection unit 210 is configured to detect the baggage of the user by the behavior of the movable part of the operation terminal 100. The above configuration for driving the table 135 is an example, and another part of the operation terminal 100 may be moved to detect the baggage of the user.

### (Flow of Operation)

Next, a flow of operation of the baggage detection unit 210 described above will be described with reference to FIG. 7. FIG. 7 is a flowchart illustrating the flow of the operation of the baggage detection unit according to the third example embodiment.

As illustrated in FIG. 7, first, the baggage detection unit 210 according to the third example embodiment drives the lifting mechanism 122 of the operation terminal 100 (step S301). The baggage detection unit 210 may drive the lifting mechanism 122 after confirming that the user has finished using the operation terminal 100 (e.g., after the user is no longer detected).

Subsequently, the baggage detection unit 210 determines whether or not the lifting mechanism 122 is driven to the movable limit (step S302).
When the lifting mechanism 122 is driven to the movable limit (step S302: YES), the baggage detection unit 210 determines that there is no baggage placed on the operation terminal 100 (more specifically, in a movable range of the movable part driven by the lifting mechanism 122) (step S303). In this case, the baggage detection unit 210 does not detect the contact position of the user.

On the other hand, when the lifting mechanism 122 is not driven to the movable limit (step S302: NO), the baggage detection unit 210 determines that there is the baggage of the user placed on the operation terminal 100 (step S304). In this instance, the baggage detection unit 210 detects a position corresponding to the detected baggage, as the contact position of the user (step S305).

The position detected as the contact position may be, for example, a position in which the baggage is in contact with the operation terminal 100. Alternatively, a position that the user may touch when placing the baggage, may be detected as the contact position of the user.

### (Technical Effect)

Next, a technical effect obtained by the information processing system 10 according to the third example embodiment will be described.

As described in FIG. 6 and FIG. 7, in the information processing system 10 according to the third example embodiment, the movable part of the operation terminal 100 is driven, by which it is detected whether or not the baggage of the user is placed on the operation terminal 100. In this way, it is possible to detect the contact position of the user in accordance with the position of the baggage of the user. Therefore, it is possible to properly identify the sanitization part. In the configuration of the operation terminal 100 illustrated in FIG. 5, for example, an area under the table 135 is a blind spot of each of the cameras. Even in a place where the camera or the like hardly detects the baggage, the baggage detection unit 210 according to the present example embodiment is allowed to detect the presence of the baggage.

### <Fourth Example Embodiment>

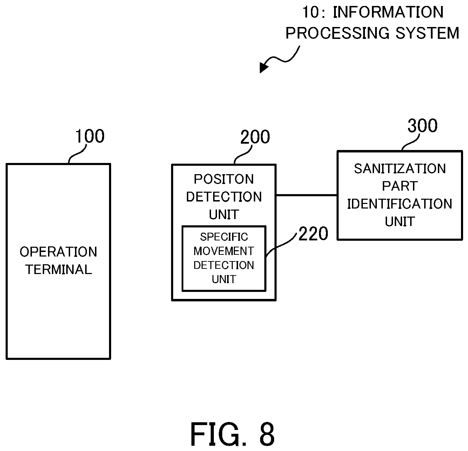

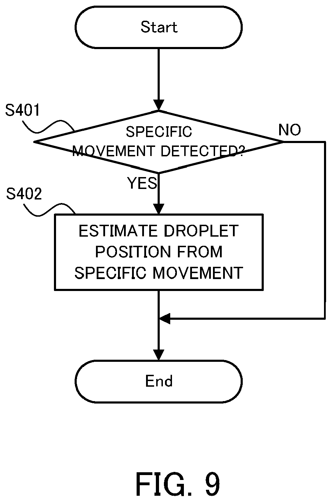

The information processing system 10 according to a fourth example embodiment will be described with reference to FIG. 8 and FIG. 9. The fourth example embodiment is partially different from the first to third example embodiments only in the configuration and operation, and may be the same as the first to third example embodiments in other parts. For this reason, a description of a part that overlaps with the example embodiments described above will be omitted below.

### (Functional Configuration)

First, a functional configuration of the information processing system 10 according to the fourth example embodiment will be described with reference to FIG. 8. FIG. 8 is a block diagram illustrating the functional configuration of the information processing system according to the fourth example embodiment. In FIG. 8, the same components as those illustrated in FIG. 2 carry the same reference numerals.

As illustrated in FIG. 8, the information processing system 10 according to the fourth example embodiment includes the operation terminal 100, the position detection unit 200, and the sanitization part identification unit 300. In particular, the position detection unit 200 according to the fourth example embodiment includes a specific movement detection unit 220.

The specific movement detection unit 220 is configured to detect the droplet position by detecting a specific movement of the user. Here, the "specific movement" is a movement in which the user is likely to exhale droplets of saliva, and an example thereof includes, for example, sneezing, speaking or the like. The specific movement detection unit 220 may detect the specific movement of the user, by using each of the cameras provided in the operation terminal 100 (e.g., the monitor camera 116, the face authentication cameras 118A and 118B, the cameras close at hand 121R and 121L), for example. Alternatively, the specific movement detection unit 220 may detect the specific movement, by using a microphone provided in the operation terminal 100 (e.g., it may detect the specific movement from a sound of sneezing or a voice when the user speaks).

### (Flow of Operation)

Next, with reference to FIG. 9, a flow of operation of the specific movement detection unit 220 will be described. FIG. 9 is a flowchart illustrating the flow of the operation of the specific movement detection unit according to the fourth example embodiment.

As illustrated in FIG. 9, first, the specific movement detection unit 220 according to the fourth example embodiment determines whether or not the specific movement of the user is detected (step S401). The specific movement detection unit 220 may always detect the specific movement while the user uses the operation terminal 100. Alternatively, the specific movement detection unit 220 may not always detect the specific movement, but may detect the specific movement at each predetermined period (e.g., every few seconds), for example.

When the specific movement of the user is detected (step S401: YES), the specific movement detection unit 220 estimates the droplet position from the specific movement (step S402). That is, the specific movement detection unit 220 estimates in which part of the operation terminal 100 the droplets are exhaled due to the specific movement are attached. The existing techniques/technologies can be adopted, as appropriate, to a method of estimating the droplet position from the specific movement, but the droplet position may be estimated from a direction of a face, a direction of a mouth, a volume of a voice or the loke, when the specific movement is performed, for example.

On the other hand, when the specific movement of the user is not detected (step S401: NO), the step S402 is omitted. That is, the specific movement detection unit 220 does not estimate the droplet position.

The specific movement detection unit 220 may operate to capture an image with a camera or the like when the specific movement is performed, and to store the image. The image captured in this manner may be used to present the sanitization part in an easier-to-understand manner, for example. Specifically, when an image of the user sneezing is displayed, it is easier to estimate around which area the droplets are exhaled, from the direction of the face at that time or the like. The image may be pixelated or blurred, or masked with a bar across the eyes in consideration of privacy of the user, for example. The specific movement detection unit 220 may store a video when the user performs the specific movement, instead of the image.

### (Technical Effect)

Next, a technical effect obtained by the information processing system 10 according to the fourth example embodiment will be described.

As described in FIG. 8 and FIG. 9, in the information processing system 10 according to the fourth example embodiment, the specific movement of the user is detected, and the droplet position is estimated from the specific movement. In this way, it is possible to easily detect the droplet position. As a result, it is possible to properly identify the sanitization part of the operation terminal 100.

### <Fifth Example Embodiment>

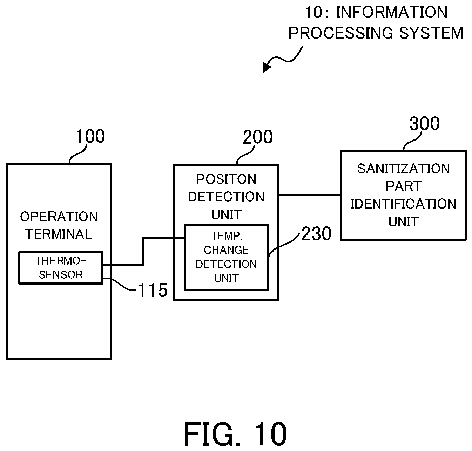

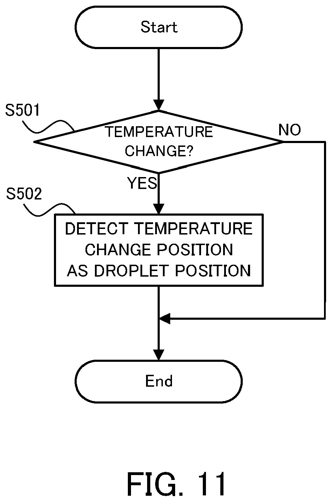

The information processing system 10 according to a fifth example embodiment will be described with reference to FIG. 10 and FIG. 11. The fifth example embodiment is partially different from the first to fourth example embodiments only in the configuration and operation, and may be the same as the first to fourth example embodiments in other parts. For this reason, a description of a part that overlaps with the example embodiments described above will be omitted below.

### (Functional Configuration)

First, a functional configuration of the information processing system 10 according to the fifth example embodiment will be described with reference to FIG. 10. FIG. 10 is a block diagram illustrating the functional configuration of the information processing system according to the fifth example embodiment. In FIG. 10, the same components as those illustrated in FIG. 2 carry the same reference numerals.

As illustrated in FIG. 10, the information processing system 10 according to the fifth example embodiment includes the operation terminal 100, the position detection unit 200, and the sanitization part identification unit 300. In particular, the position detection unit 200 according to the fifth example embodiment includes a temperature change detection unit 230.

The temperature change detection unit 230 is configured to detect a part in which there is a temperature change in the operation terminal 100, by using the thermosensor 115 of the operation terminal 100, and to detect the part in which there is a temperature change, as the droplet position. For example, it is considered that in a part in which the droplets are attached in the operation terminal 100, the temperature is temporarily lowered due to the heat of vaporization of the droplets. The temperature change detection unit 230 is configured to detect the droplet position, by detecting the temperature change caused by the attachment of the droplets. The temperature change detection unit 230 may detect the temperature change by using another thermosensor, in addition to or instead of the thermosensor 115 of the operation terminal 100.

### (Flow of Operation)

Next, with reference to FIG. 11, a flow of operation of the temperature change detection unit 230 will be described. FIG. 11 is a flowchart illustrating the flow of the operation of the temperature change detection unit according to the fifth example embodiment.

As illustrated in FIG. 11, first, the temperature change detection unit 230 according to the fifth example embodiment determines whether or not there is a temperature change in the operation terminal 100 (step S501). Whether or not there is a temperature change may be determined by using a predetermined threshold, for example. Specifically, it may be determined that there is a temperature change when a change width of the temperature is greater than a predetermined threshold, and it may be determined that there is no temperature change when the change width of the temperature is less than or equal to the predetermined threshold.

When the temperature change in the operation terminal 100 is detected (step S501: YES), the temperature change detection unit 230 detects the part in which there is a temperature change, as the droplet position (step S502). On the other hand, when the temperature change in the operation terminal 100 is not detected (step S501: NO), the step S502 is omitted. That is, the temperature change detection unit 230 does not detect the droplet position.

### (Technical Effect)

Next, a technical effect obtained by the information processing system 10 according to the fifth example embodiment will be described.

As described in FIG. 10 and FIG. 11, in the information processing system 10 according to the fifth example embodiment, the part in which there is a temperature change in the operation terminal 100 is detected as the droplet position. In this way, it is possible to accurately detect the droplet position. As a result, it is possible to properly identify the sanitization part of the operation terminal 100.

### <Sixth Example Embodiment>

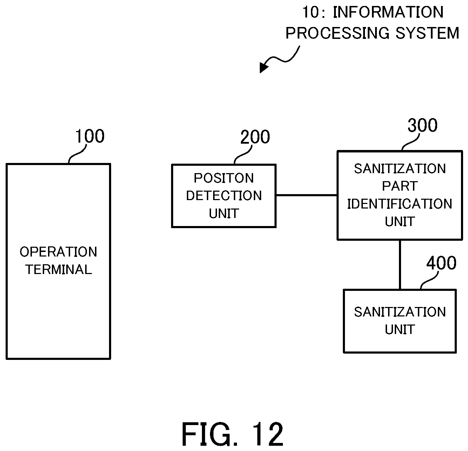

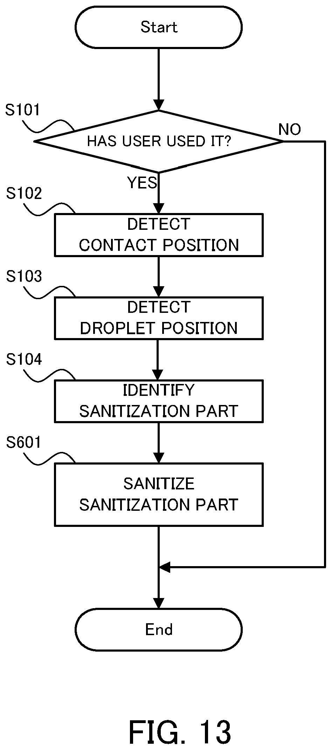

The information processing system 10 according to a sixth example embodiment will be described with reference to FIG. 12 and FIG. 13. The sixth example embodiment is partially different from the first to fifth example embodiments only in the configuration and operation, and may be the same as the first to fifth example embodiments in other parts. For this reason, a description of a part that overlaps with the example embodiments described above will be omitted below.

### (Functional Configuration)

First, a functional configuration of the information processing system 10 according to the sixth example embodiment will be described with reference to FIG. 12. FIG. 12 is a block diagram illustrating the functional configuration of the information processing system according to the sixth example embodiment. In FIG. 12, the same components as those illustrated in FIG. 2 carry the same reference numerals.

As illustrated in FIG. 12, the information processing system 10 according to the sixth example embodiment includes the operation terminal 100, the position detection unit 200, the sanitization part identification unit 300, and a sanitization unit 400. That is, the information processing system 10 according to the sixth example embodiment further includes the sanitization unit 400, in addition to the configuration in the first example embodiment (see FIG. 2).

The sanitization unit 400 is configured to sanitize the sanitization part identified by the sanitization part identification unit 300. Although there is no particular limitation on a specific method of sanitization, the sanitization unit 400 may sanitize the sanitization part by spraying thereon a sanitizer or an antiseptic solution, for example. Alternatively, the sanitization unit 400 may sanitize the sanitization part by irradiating it with ultraviolet rays. A means to be used by the sanitization unit 400 may be provided in advance in the operation terminal 100. For example, the operation terminal 100 may be provided with an antiseptic solution for sanitization and a mechanism for automatically spraying the antiseptic solution. In this case, the antiseptic solution may be provided in a part that is hardly seen by the user. Specifically, the antiseptic solution may be provided in such a part that appears outside only when the lifting mechanism 122 is driven (e.g., a part of the bar 134 that is hidden by the table 135).

The sanitization unit 400 may further perform the sanitization by making the operation terminal 100 self-travel to a predetermined sanitization area (i.e., an area in which the sanitization part is automatically sanitized), when the operation terminal 100 is configured to self-travel. In this case, when the sanitization is completed, the operation terminal 100 may be automatically returned to an original position.

### (Flow of Operation)

Next, a flow of operation of the information processing system 10 according to the sixth example embodiment will be described with reference to FIG. 13. FIG. 13 is a flowchart illustrating the flow of the operation of the information processing system according to the sixth example embodiment. In FIG. 13, the same steps as those illustrated in FIG. 3 carry the same reference numerals.

As illustrated in FIG. 13, in operation of the information processing system 10 according to the sixth example embodiment, it is first detected whether or not the user has used the operation terminal 100 (step S101). When it is determined that the user has used the operation terminal 100 (step S101: YES), the position detection unit 200 detects the contact position (step S102). The position detection unit 200 also detects the droplet position (step S103).

Subsequently, the sanitization part identification unit 300 identifies the sanitization part of the operation terminal 100, on the basis of the contact position and the droplet position (step S104). Especially in the sixth example embodiment, the sanitization unit 400 sanitizes the sanitization part identified by the sanitization part identification unit 300 (step S601).

The sanitization unit 400 may perform the sanitization, after confirming that the user has left the operation terminal 100. Alternatively, the sanitization unit 400 may be performed sanitization, after confirming that there is no one in the vicinity of the operation terminal 100.

### (Technical Effect)

Next, a technical effect obtained by the information processing system 10 according to the sixth example embodiment will be described.

As described in FIG. 12 and FIG. 13, in the information processing system 10 according to the sixth example embodiment, the identified sanitization part is sanitized automatically. In this way, it is possible to reliably sanitize the identified sanitization part, and to effectively prevent the spread of infectious diseases, for example. Furthermore, since it is not necessary to perform the sanitization by hand, it is possible to reduce a burden required for the sanitization.

### <Seventh Example Embodiment>

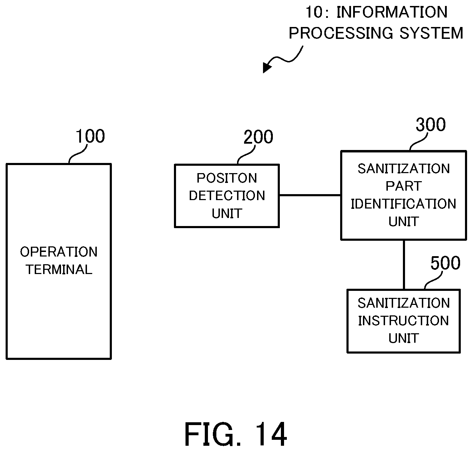

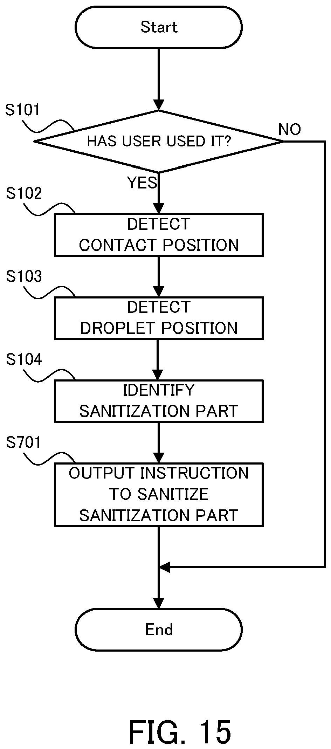

The information processing system 10 according to a seventh example embodiment will be described with reference to FIG. 14 and FIG. 15. The seventh example embodiment is partially different from the first to sixth example embodiments only in the configuration and operation, and may be the same as the first to sixth example embodiments in other parts. For this reason, a description of a part that overlaps with the example embodiments described above will be omitted below.

### (Functional Configuration)

First, a functional configuration of the information processing system 10 according to the seventh example embodiment will be described with reference to FIG. 14. FIG. 14 is a block diagram illustrating the functional configuration of the information processing system according to the seventh example embodiment. In FIG. 14, the same components as those illustrated in FIG. 2 carry the same reference numerals.

As illustrated in FIG. 14, the information processing system 10 according to the seventh example embodiment includes the operation terminal 100, the position detection unit 200, the sanitization part identification unit 300, and a sanitization instruction unit 500. That is, the information processing system 10 according to the seventh example embodiment further includes the sanitization instruction unit 500, in addition to the configuration in the first example embodiment (see FIG. 2).

The sanitization instruction unit 500 is configured to output a sanitization instruction for instructing the sanitization of the sanitization part identified by the sanitization part identification unit 300. Although there is no particular limitation on an output aspect of the sanitization instruction, the sanitization instruction unit 500 may output the sanitization instruction by using a display, for example. In this instance, an image indicating the sanitization part (e.g., an image of the entire operation terminal 100 in which the sanitization part is colored in red) may be displayed on the display. Alternatively, the sanitization instruction unit 500 may provide the sanitization instruction by irradiating the sanitization part with a spotlight. Alternatively, the sanitization instruction unit 500 may provide the sanitization instruction, by outputting audio from a speaker. In this case, the outputted audio may include a message indicating the sanitization part. An instruction target of the sanitization instruction may be the user who uses the operation terminal 100, or another user (e.g., a system administrator or manager, a staff of a facility where the system is installed, etc.).

### (Flow of Operation)

Next, a flow of operation of the information processing system 10 according to the seventh example embodiment will be described with reference to FIG. 15. FIG. 15 is a flowchart illustrating the flow of the operation of the information processing system according to the seventh example embodiment. In FIG. 15, the same steps as those illustrated in FIG. 3 carry the same reference numerals.

As illustrated in FIG. 15, in operation of the information processing system 10 according to the seventh example embodiment, it is first detected whether or not the user has used the operation terminal 100 (step S101). When it is determined that the user has used the operation terminal 100 (step S101: YES), the position detection unit 200 detects the contact position (step S102). The position detection unit 200 also detects the droplet position (step S103).

Subsequently, the sanitization part identification unit 300 identifies the sanitization part of the operation terminal 100, on the basis of the contact position and the droplet position (step S104). Especially in the seventh example embodiment, the sanitization instruction unit 500 outputs the instruction to sanitize the sanitization part identified by the sanitization part identification unit 300 (step S701).

The sanitization instruction unit 500 may output the sanitization instruction after confirming that the user has left the operation terminal 100 (in other words, after the sanitization is possible). Then, the sanitization instruction unit 500 may provide a display encouraging the user not to use the operation terminal 100, until the sanitization is performed in accordance with the instruction (e.g., a message of "Please do not use it until the sanitization is completed" or the like may be displayed on the touch panel 117).

### (Technical Effect)

Next, a technical effect obtained by the information processing system 10 according to the seventh example embodiment will be described.

As described in FIG. 14 and FIG. 15, in the information processing system 10 according to the seventh example embodiment, the sanitization instruction for the identified sanitization part is outputted. In this way, it is possible to make a staff or the like sanitize the identified sanitization part, and to effectively prevent the spread of infectious diseases, for example.

### <Eighth Example Embodiment>

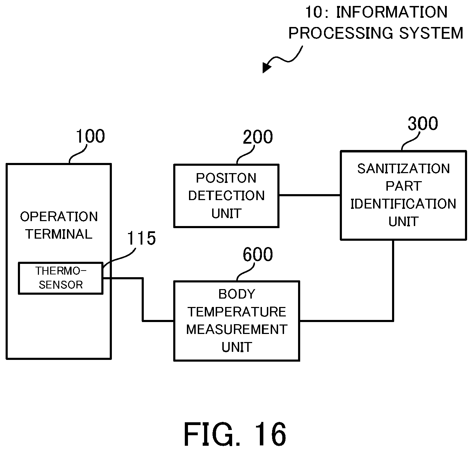

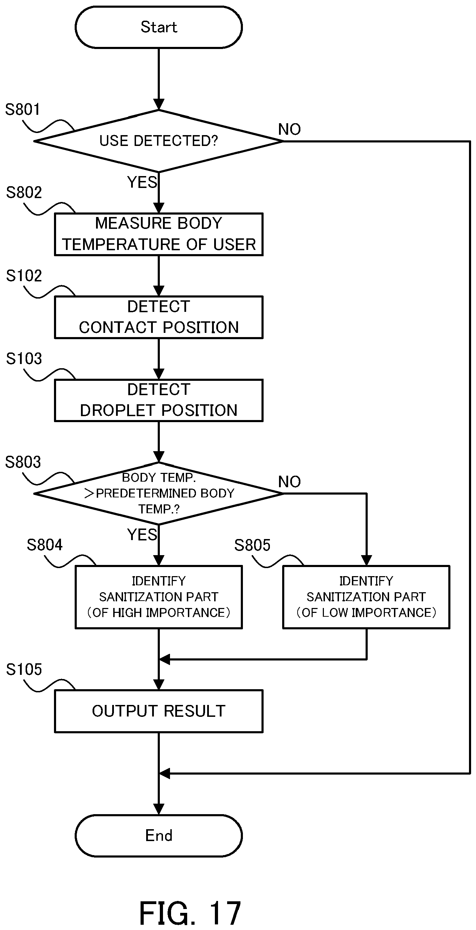

The information processing system 10 according to an eighth example embodiment will be described with reference to FIG. 16 and FIG. 17. The information processing system 10 according to the eighth example embodiment is partially different from the first to seventh example embodiments only in the configuration and operation, and may be the same as the first to seventh example embodiments in other parts. For this reason, a description of a part that overlaps with the example embodiments described above will be omitted below.

### (Functional Configuration)

First, a functional configuration of the information processing system 10 according to the eighth example embodiment will be described with reference to FIG. 16. FIG. 16 is a block diagram illustrating the functional configuration of the information processing system according to the eighth example embodiment. In FIG. 16, the same components as those illustrated in FIG. 2 carry
the same reference numerals.

As illustrated in FIG. 16, the information processing system 10 according to the eighth example embodiment includes the operation terminal 100, the position detection unit 200, the sanitization part identification unit 300, and a body temperature measurement unit 600. That is, the information processing system 10 according to the eighth example embodiment further includes the body temperature measurement unit 600, in addition to the configuration in the first example embodiment (see FIG. 2).

The body temperature measurement unit 600 is configured to measure a body temperature of the user who uses the operation terminal 100, by using the thermosensor 115 of the operation terminal 100. The body temperature measurement unit 600 may detect a temperature change by using another thermosensor, in addition to or instead of the thermosensor 115 of the operation terminal 100. Information about the body temperature of the user measured by the body temperature measurement unit 600 is configured to be outputted to the sanitization part identification unit 300.

The sanitization part identification unit 300 according to the eighth example embodiment is configured to set importance of the sanitization part, on the basis of the body temperature of the user measured by the body temperature measurement unit 600. The " importance" here indicates a degree of necessity of the sanitization. For example, as the importance increases, a more careful or quick sanitization is required. A specific method of setting the importance will be described in detail in the following description of operation.

### (Flow of Operation)

Next, a flow of operation of the information processing system 10 according to the eighth example embodiment will be described with reference to FIG. 17. FIG. 17 is a flowchart illustrating the flow of the operation of the information processing system according to the eighth example embodiment. In FIG. 17, the same steps as those illustrated in FIG. 3 carry the same reference numerals.

As illustrated in FIG. 17, in operation of the information processing system 10 according to the eighth example embodiment operates, first, the user who uses the operation terminal 100 is detected (step S801). When the user who uses the operation terminal 100 cannot be detected (step S801: NO), the subsequent processing steps may be omitted.

On the other hand, when the user who uses the operation terminal 100 is detected (step S801: YES), the body temperature measurement unit 600 measures the body temperature of the user (step S802). Then, the position detection unit 200 detects the contact position (step S102). The position detection unit 200 also detects the droplet position (step S103). The step of measuring the body temperature (i.e., the step S802) may be performed in parallel with the step of detecting the contact position (i.e., the step S102) or the step of detecting the droplet position (i.e., the step S103).

Subsequently, especially in the eighth example embodiment, the sanitization part identification unit 300 determines whether or not the body temperature of the user exceeds a predetermined body temperature (step S803). The "predetermined body temperature" here is a threshold for determining that the user is likely to be infected by infectious diseases (in other words, a threshold for determining a fever of the user), and may be set as 37.0 degrees, for example.

When the body temperature of the user exceeds the predetermined body temperature (step S803: YES), the sanitization part identification unit 300 identifies the sanitization part to be of high importance (step S804). On the other hand, when the body temperature of the user does not exceed the predetermined body temperature (step S803: NO), the sanitization part identification unit 300 identifies the sanitization part to be of low importance (step S805). Then, the sanitization part identification unit 300 outputs information about the identified sanitization part (including the importance) (step S105).

As described in the sixth example embodiment, when the information processing system 10 includes the sanitization unit 400, the sanitization may be performed in the sanitization part of high importance, more carefully than in the sanitization part of low importance. For example, the antiseptic solution may be sprayed more than usual. Furthermore, as described in the seventh example embodiment, when the information processing system 10 includes the sanitization instruction unit 500, the instruction to perform the sanitization more carefully may be outputted in the sanitization part of high importance, in comparison with the instruction in the sanitization part of low importance. For example, the color of the display may be changed to a conspicuous color (e.g., a dark red color), or intensity of the spotlight may be increased.

### (Technical Effect)

Next, a technical effect obtained by the information processing system 10 according to the eighth example embodiment will be described.

As described in FIG. 16 and FIG. 17, in the information processing system 10 according to the eighth example embodiment, the importance of the sanitization part is set in accordance with the body temperature of the user. In this way, after the use by the user who is highly likely to be infected with infectious diseases, the sanitization is performed more carefully than usual. Consequently, it is possible to effectively prevent the spread of infectious diseases, for example.

A processing method in which a program for allowing the configuration in each of the example embodiments to operate to realize the functions of each of the example embodiments is recorded on a recording medium, and in which the program recorded on the recording medium is read as a code and executed on a computer, is also included in the scope of each of the example embodiments. That is, a computer-readable recording medium is also included in the range of each of the example embodiments. Not only the recording medium on which the above-described program is recorded, but also the program itself is also included in each example embodiment.

The recording medium to use may be, for example, a floppy disk (registered trademark), a hard disk, an optical disk, a magneto-optical disk, a CD-ROM, a magnetic tape, a nonvolatile memory card, or a ROM. Furthermore, not only the program that is recorded on the recording medium and executes processing alone, but also the program that operates on an OS and executes processing in cooperation with the functions of expansion boards and another software, is also included in the scope of each of the example embodiments.

This disclosure is not limited to the examples described above and is allowed to be changed, if desired, without departing from the essence or spirit of this disclosure which can be read from the claims and the entire specification. An information processing system, an information processing method, and a computer program with such changes are also intended to be within the technical scope of this disclosure.

### <Supplementary Notes>

The example embodiments described above may be further described as, but not limited to, the following Supplementary Notes.

### (Supplementary Note 1)

An information processing system according to Supplementary Note 1 is an information processing system including: a terminal used by a user; a detection unit that detects at least one of a position of contact by the user and a position in which droplets of saliva of the user are attached, in the terminal; and an identification unit that identifies a sanitization part to be sanitized of the terminal, on the basis of the at least one of the positions.

### (Supplementary Note 2)

An information processing system according to Supplementary Note 2 is the information processing system according to Supplementary Note 1, wherein the detection unit detects the position of contact by the user, by using at least one of a camera, a touch panel, and a weight sensor that are provided in the terminal.

### (Supplementary Note 3)

An information processing system according to Supplementary Note 3 is the information processing system according to Supplementary Note 1 or 2, wherein the terminal includes a movable part, and the detection unit determines whether or not a baggage of the user is placed on the terminal, on the basis of behavior when the movable part is driven, and detects a part in which the baggage is placed, as the position of contact by the user.

### (Supplementary Note 4)

An information processing system according to Supplementary Note 4 is the information processing system according to any one of Supplementary Notes 1 to 3, wherein the detection unit detects the position in which droplets of saliva of the user are attached, by detecting a specific movement of the user.

### (Supplementary Note 5)

An information processing system according to Supplementary Note 5 is the information processing system according to any one of Supplementary Notes 1 to 4, wherein the detection unit detects a part in which there is a temperature change in the terminal, as the position in which droplets of saliva of the user are attached.

### (Supplementary Note 6)

An information processing system according to Supplementary Note 6 is the information processing system according to any one of Supplementary Notes 1 to 5, further including a sanitization unit that sanitizes the sanitization part.

### (Supplementary Note 7)

An information processing system according to Supplementary Note 7 is the information processing system according to any one of Supplementary Notes 1 to 6, further including a sanitization instruction unit that outputs a sanitization instruction to make the user to sanitize the sanitization part.

### (Supplementary Note 8)

An information processing system according to Supplementary Note 8 is the information processing system according to any one of Supplementary Notes 1 to 7, further including a body temperature measurement unit that measures a body temperature of the user, wherein the identification unit sets the sanitization part identified when the sanitization part is used by the user with a high body temperature, to be of high importance of the sanitization, as compared with the sanitization part identified when the sanitization part is used by the user with a low body temperature.

### (Supplementary Note 9)

An information processing method according to Supplementary Note 9 is an information processing method using a terminal used by a user, the information processing method including: detecting one of a position of contact by the user and a position in which droplets of saliva of the user are attached, in the terminal; and identifying a sanitization part to be sanitized of the terminal, on the basis of the at least one of the positions.

### (Supplementary Note 10)

A computer program according to Supplementary Note 10 is a computer program that allows a computer to execute an information processing method using a terminal used by a user, the information processing method including: detecting one of a position of contact by the user and a position in which droplets of saliva of the user are attached, in the terminal; and identifying a sanitization part to be sanitized of the terminal, on the basis of the at least one of the positions.

### (Supplementary Note 11)

A recording medium described in Supplementary Note 11 is a recording medium on which the computer program according to Supplementary Note 10 is recorded.

### Description of Reference Codes

10 Information processing system
11 Processor
100 Operation terminal
115 Thermosensor
122 Lifting mechanism
200 Position detection unit
210 Baggage detection unit
220 Specific movement detection unit
230 Temperature change detection unit
300 Sanitization part identification unit
400 Sanitization unit
500 Sanitization instruction unit
600 Body temperature measurement unit

## Claims

1. An information processing system comprising:
a terminal used by a user;
a detection unit that detects at least one of a position of contact by the user and a position in which droplets of saliva of the user are attached, in the terminal; and
an identification unit that identifies a sanitization part to be sanitized of the terminal, on the basis of the at least one of the positions.

2. The information processing system according to claim 1, wherein the detection unit detects the position of contact by the user, by using at least one of a camera, a touch panel, and a weight sensor that are provided in the terminal.

3. The information processing system according to claim 1 or 2, wherein
the terminal includes a movable part, and
the detection unit determines whether or not a baggage of the user is placed on the terminal, on the basis of behavior when the movable part is driven, and detects a part in which the baggage is placed, as the position of contact by the user.

4. The information processing system according to any one of claims 1 to 3, wherein the detection unit detects the position in which droplets of saliva of the user are attached, by detecting a specific movement of the user.

5. The information processing system according to any one of claims 1 to 4, wherein the detection unit detects a part in which there is a temperature change in the terminal, as the position in which droplets of saliva of the user are attached.

6. The information processing system according to any one of claims 1 to 5, further comprising a sanitization unit that sanitizes the sanitization part.

7. The information processing system according to any one of claims 1 to 6, further comprising a sanitization instruction unit that outputs a sanitization instruction to make the user to sanitize the sanitization part.

8. The information processing system according to any one of claims 1 to 7, further comprising a body temperature measurement unit that measures a body temperature of the user, wherein
the identification unit sets the sanitization part identified when the sanitization part is used by the user with a high body temperature, to be of high importance of the sanitization, as compared with the sanitization part identified when the sanitization part is used by the user with a low body temperature.

9. An information processing method using a terminal used by a user,
the information processing method comprising:
detecting one of a position of contact by the user and a position in which droplets of saliva of the user are attached, in the terminal; and
identifying a sanitization part to be sanitized of the terminal, on the basis of the at least one of the positions.

10. A computer program that allows a computer to execute an information processing method using a terminal used by a user,
the information processing method including:
detecting one of a position of contact by the user and a position in which droplets of saliva of the user are attached, in the terminal; and
identifying a sanitization part to be sanitized of the terminal, on the basis of the at least one of the positions.
